(19) Europäisches Patentamt
European Patent Office
Office européen des brevets

(11) Veröffentlichungsnummer: **0 308 608 A1**

(12) **EUROPÄISCHE PATENTANMELDUNG**

(21) Anmeldenummer: **88111484.7**

(22) Anmeldetag: **16.07.88**

(51) Int. Cl.4: **C07D 401/12 , A61K 31/50 , C07D 237/04 , //A61K31/445**

(30) Priorität: **28.07.87 DE 3724909**

(43) Veröffentlichungstag der Anmeldung: **29.03.89 Patentblatt 89/13**

(84) Benannte Vertragsstaaten: **AT BE CH DE ES FR GB GR IT LI NL SE**

(71) Anmelder: **BAYER AG**
**Konzernverwaltung RP Patentabteilung**
**D-5090 Leverkusen 1 Bayerwerk(DE)**

(72) Erfinder: **Franckowiak, Gerhard, Dr.**
**Henselweg 10**
**D-5600 Wuppertal 1(DE)**
Erfinder: **Bechem, Martin, Dr.**
**Obere Bergerheide 4**
**D-5600 Wuppertal 1(DE)**
Erfinder: **Kayser, Michael, Dr.**
**Walter-Flex-Strasse 18**
**D-5090 Leverkusen 1(DE)**
Erfinder: **Gross, Rainer, Prof. Dr.**
**Platzhofstrasse 23**
**D-5600 Wuppertal 1(DE)**
Erfinder: **Schramm, Matthias, Dr.**
**Paffrather Strasse 38**
**D-5000 Koeln 80(DE)**
Erfinder: **Seuter, Friedel, Dr.**
**Moospfad 16**
**D-5600 Wuppertal 1(DE)**
Erfinder: **Perzborn, Elisabeth, Dr.**
**Am Tescher Busch 13**
**D-5600 Wuppertal 11(DE)**
Erfinder: **Thomas, Günther, Dr.**
**Via Campogallo 21/4**
**I-20020 Arese (Mi)(IT)**

(54) **Neue substituierte Nitro-Dihydropyridine, Verfahren zu ihrer Herstellung und ihre Verwendung.**

(57) Die Erfindung betrifft neue substituierte Nitro-Dihydropyridine der allgemeinen Formel I

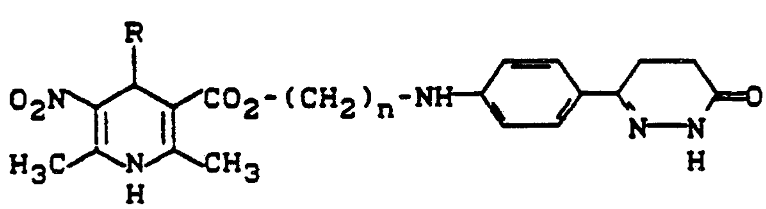

(I),

in welcher R und n die in der Beschreibung angegebene Bedeutung haben, mehrere Verfahren zu ihrer

EP 0 308 608 A1

Herstellung und ihre Verwendung in Arzneimitteln, insbesondere zur Bekämpfung von Kreislauferkrankungen und Thrombosen.

## Neue substituierte Nitro-Dihydropyridine, Verfahren zu ihrer Herstellung und ihre Verwendung

Die Erfindung betrifft neue substituierte Nitro-Dihydropyridine, mehrere Verfahren zu ihrer Herstellung und ihre Verwendung in Arzneimitteln, insbesondere zur Bekämpfung von Kreislauferkrankungen und Thrombosen.

Die vorliegende Erfindung betrifft neue substituierte Nitro-Dihydropyridine der allgemeinen Formel (I)

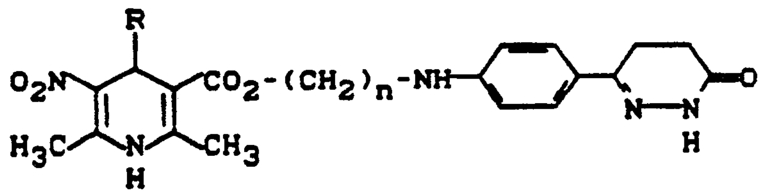

(I),

in welcher
R - für Aryl mit 6 bis 12 Kohlenstoffatomen steht, das bis zu 4-fach gleich oder verschieden substituiert sein kann durch Halogen, Nitro, Cyano, Alkyl, Alkoxy, Alkylthio mit jeweils bis zu 6 Kohlenstoffatomen, Trifluormethyl, Trifluormethoxy, Difluormeth oxy, Trifluormethylthio, Difluormethylendioxy, Trifluorethylendioxy, Tetrafluorethylendioxy oder durch gegebenenfalls durch Halogen, Nitro, Cyano, Trifluormethyl, Alkyl mit bis zu 4 Kohlenstoffatomen oder Alkoxy mit bis zu 4 Kohlenstoffatomen substituiertes Benzyl, Benzyloxy oder Benzylthio, oder
- für einen gegebenenfalls durch Halogen, Phenyl oder Alkyl mit bis zu 4 Kohlenstoffatomen substituierten Heterocyclus aus der Reihe Pyrryl, Thienyl, Furyl, Pyrazolyl, Imidazolyl, Oxazolyl, Thiazolyl, Pyridyl, Pyrimidyl, Pyridazinyl, Chinolyl, Benzoxadiazolyl, Chromenyl oder Thiochromenyl steht
und
n - für eine Zahl 1 bis 12 steht
und deren physiologisch unbedenkliche Salze.

Bevorzugt sind Verbindungen der allgemeinen Formel (I),
in welcher
R- für Phenyl oder Naphthyl steht, das bis zu 3-fach gleich oder verschieden substituiert sein kann durch Fluor, Chlor, Brom, Nitro, Cyano, Alkyl, Alkoxy, Alkylthio mit jeweils bis zu 4 Kohlenstoffatomen, Trifluormethyl, Trifluormethoxy, Trifluormethylthio, durch gegebenenfalls durch Nitro, Fluor, Chlor, Brom, Trifluormethyl, Methyl oder Methoxy substituiertes Benzyloxy oder Benzylthio, oder
- für einen gegebenenfalls durch Methyl, Fluor, Chlor, Brom oder Phenyl substituierten Heterocyclus aus der Reihe Thienyl, Furyl, Pyridyl, Pyrimidyl, Chinolyl, Benzoxadiazolyl oder Thiochromenyl steht
und
n - für eine Zahl 1 bis 10 steht
und deren physiologisch unbedenkliche Salze.

Besonders bevorzugt sind Verbindungen der allgemeinen Formel (I),
in welcher
R - für Phenyl steht, das bis zu 2-fach gleich oder verschieden substituiert sein kann durch Fluor, Chlor, Nitro, Cyano, Alkyl, Alkoxy mit jeweils bis zu 4 Kohlenstoffatomen, Trifluormethyl, Benzyloxy oder Benzylthio, oder
- für Thienyl, Furyl, Pyridyl, Benzoxadiazolyl steht, oder
- für einen Heterocyclus der Formel

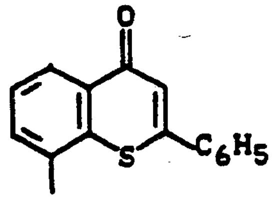

steht,

und
n - für eine Zahl 2 bis 8 steht
und deren physiologisch unbedenkliche Salze.

Physiologisch unbedenkliche Salze sind Salze der erfindungsgemäßen Verbindungen mit anorganischen oder organischen Säuren. Hierzu gehören bevorzugt anorganische Säuren wie Halogenwasserstoffsäuren, bevorzugt Salzsäure oder Bromwasserstoffsäure, oder Schwefelsäure, oder Phosphorsäure, oder organische Carbonsäuren oder Sulfonsäuren wie beispielsweise Ameisensäure, Essigsäure, Propionsäure, Maleinsäure, Fumarsäure, Äpfelsäure, Zitronensäure, Weinsäure, Milchsäure, Benzoesäure, Methansulfonsäure, Ethansulfonsäure, Benzolsulfonsäure, Naphthalindisulfonsäure oder Toluolsulfonsäure.

Die erfindungsgemäßen Verbindungen existieren in stereoisomeren Formen, die sich entweder wie Bild und Spiegelbild (Enantiomere) oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen (vgl. E.L. Eliel, Stereochemistry of Carbon Compounds, McGraw Hill, 1962).

Die erfindungsgemäßen Verbindungen der allgemeinen Formel (I) werden hergestellt,
indem man

[A] Aldehyde der allgemeinen Formel (II)

R-CHO (II)

in welcher
R die oben angegebene Bedeutung hat
und Nitroaceton der Formel (III)

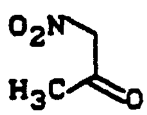

(III)

oder deren Knoevenagel-Kondensationsprodukte (Ylidenverbindungen) der allgemeinen Formel (IV)

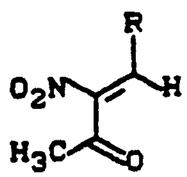

(IV)

in welcher
R die oben angegebene Bedeutung hat,
mit Enaminen der allgemeinen Formel (V)

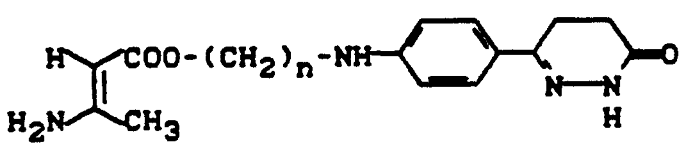

(V)

in welcher
n die oben angegebene Bedeutung hat,
in Gegenwart inerter Lösemittel umsetzt,
oder indem man

[B] Aldehyde der allgemeinen Formel (II) und Ketone der allgemeinen Formel (VI)

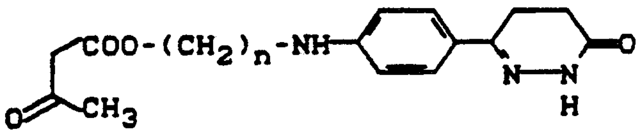

(VI)

in welcher
n die oben angegebene Bedeutung hat,
oder deren Knoevenagel-Kondensationsprodukte (Ylidenverbindungen) der allgemeinen Formel (VII)

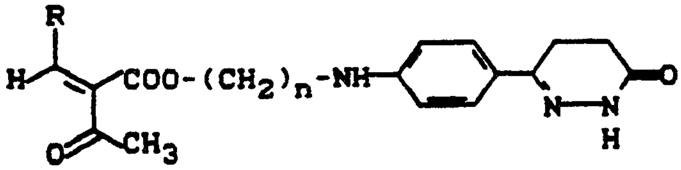

(VII)

in welcher
R und n die oben angegebene Bedeutung haben,
mit dem Enamin (Nitroaceton/Ammoniak-Additionsverbindung) der Formel (VIII),

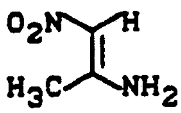

(VIII)

in Gegenwart inerter Lösemittel umsetzt,
oder indem man

[C] 1,4-Dihydropyridincarbonsäureester der allgemeinen Formel (IX)

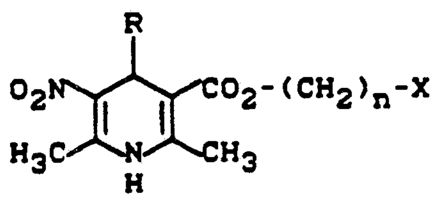

(IX)

in welcher
R und n die oben angegebene Bedeutung haben
und
X - eine nucleofuge Gruppe wie beispielsweise Bromid, Iodid, Mesylat, Triflat oder Tosylat darstellt,
mit dem Anilin der Formel (X)

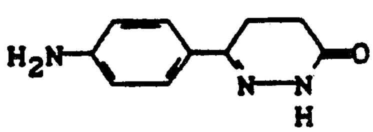

(X)

in Gegenwart einer Hilfsbase in einem inerten Lösemittel umsetzt,
oder indem man

[D] 1,4-Dihydropyridincarbonsäuren der allgemeinen Formel (XI)

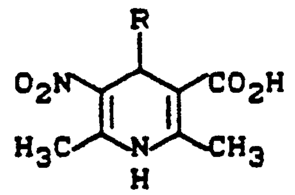

(XI)

in welcher
R die angegebene Bedeutung hat,
nach üblichen Methoden der Veresterung von Carbonsäuren (z.B. über das Carbonsäurechlorid oder -imidazolid oder in Gegenwart von Dicyclohexylcarbodiimid) mit dem Alkohol der Formel (XII)

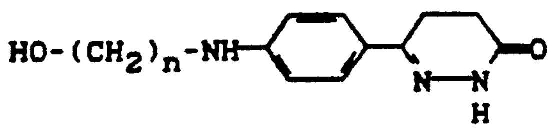

(XII)

in welcher
n die angegebene Bedeutung hat,
verestert.

Je nach Art der eingesetzten Ausgangsverbindungen lassen sich die Verfahrensvarianten A bis D durch folgende Formelschemata verdeutlichen:

[A]

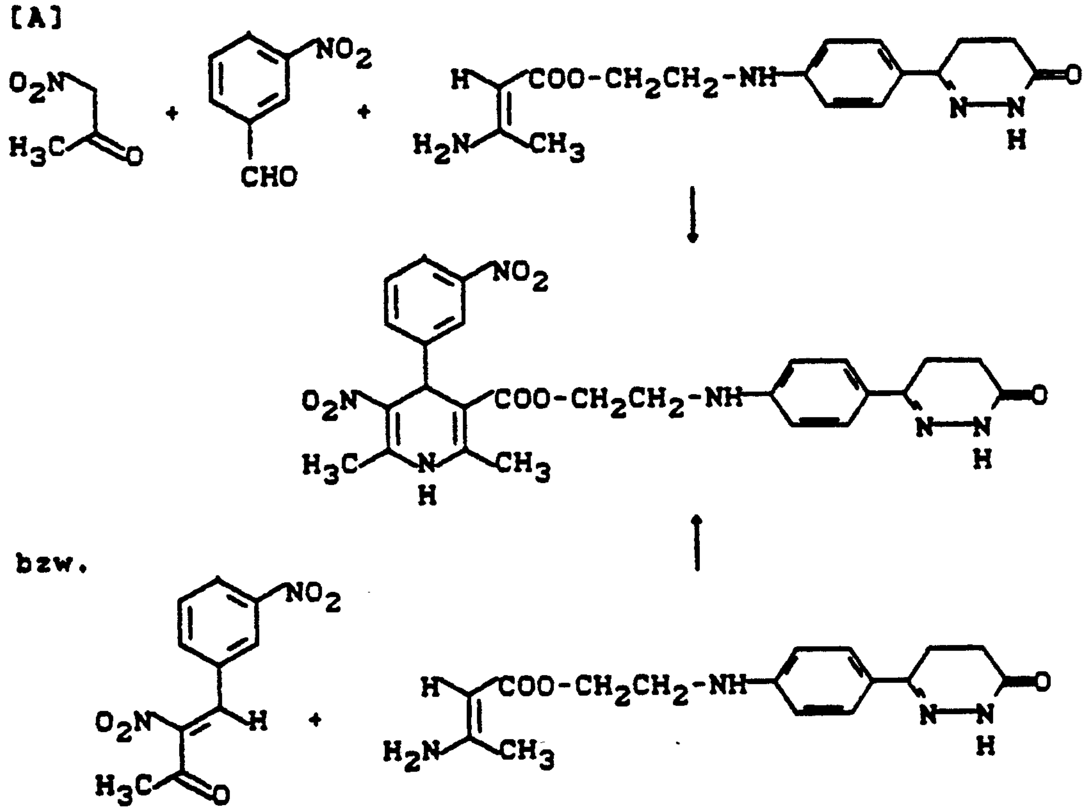

[B]

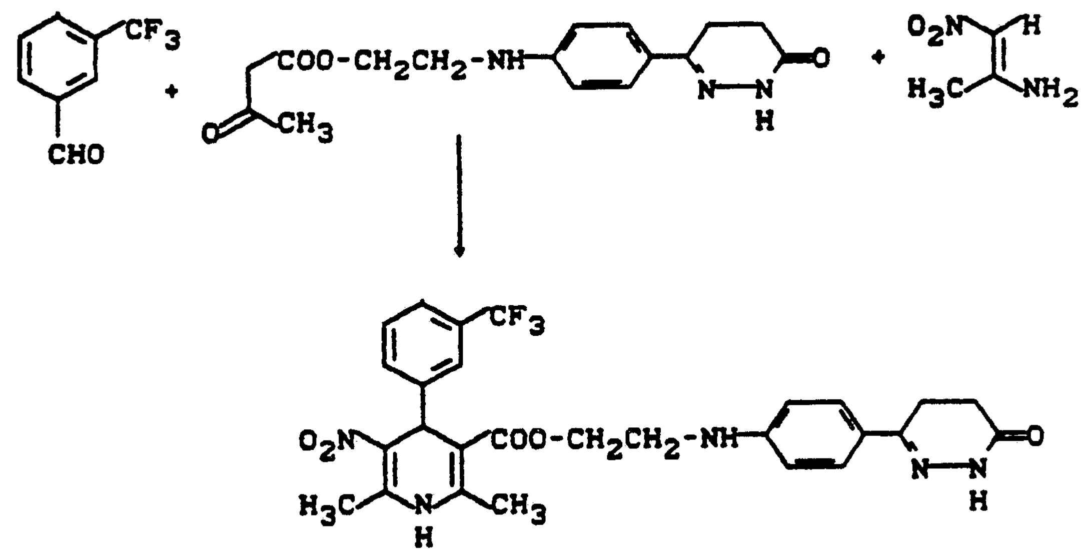

bzw.

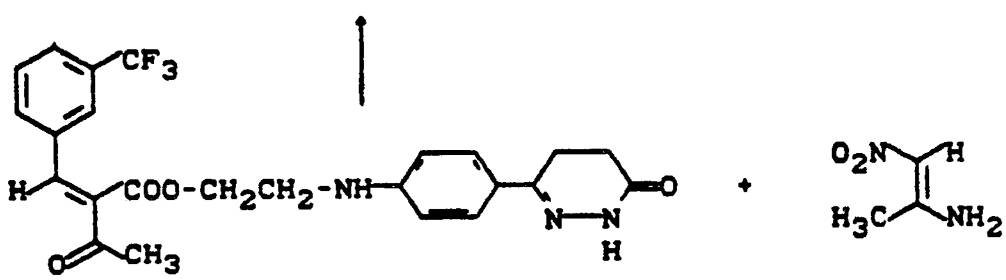

[C]

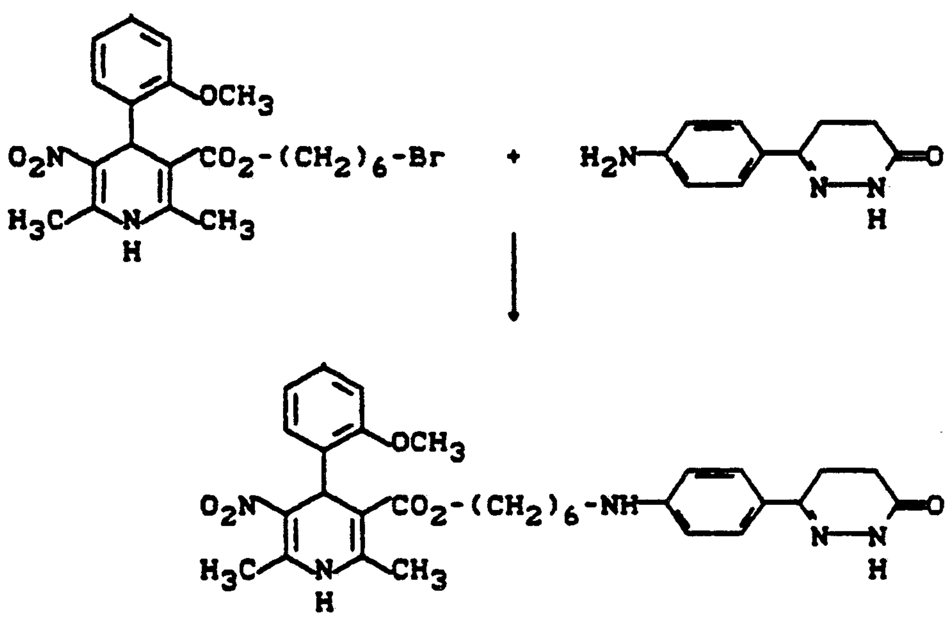

[D]

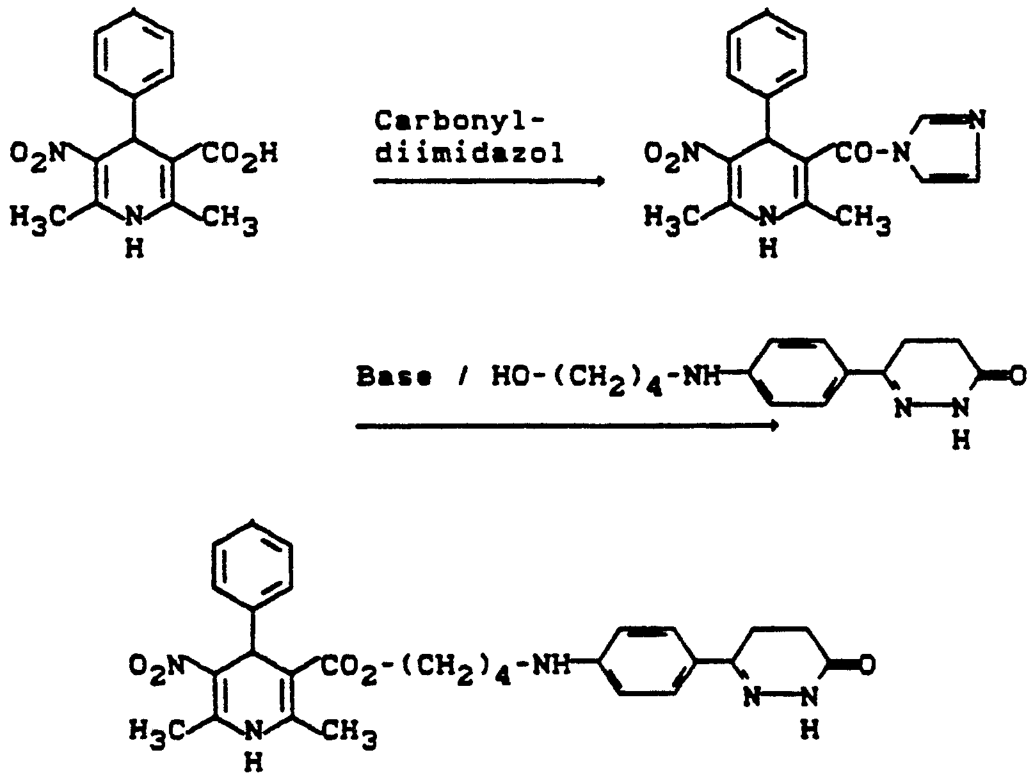


Die als Ausgangsstoffe eingesetzten Aldehyde der allgemeinen Formel (II) sind bekannt oder können nach bekannten Method hergestellt werden [E. Mosettig, Organic Reactions III, 218 (1954); Chemical Abstracts 59, 13929 (1963)].

Das Keton der Formel (III) ist bekannt [N. Lewy, C.W. Scaife, J. Chem. Soc. (London) 1946, 1100; C.D. Hurd, M.E. Nilson J. Org. Chem. 20, 927 (1955); G.F. Field, W.J. Zally Synthesis 1979, 295].

Das Enamin (Nitroaceton/Ammoniak-Additionsverbindung) der Formel (VIII) ist bekannt [H. Boehme, K.-H. Weisel Arch. Pharm. 310, 30 (1977)].

Die Knoevenagel-Kondensationsprodukte der allgemeinen Formel (IV) sind bekannt oder können nach bekannten Methoden hergestellt werden [A. Dornow, W. Sassenberg, Liebigs Ann. Chem. 602, 14 (1957)].

Die Ketone der allgemeinen Formel (VI) sind neu. Sie werden nach bekannten Methoden hergestellt [z.B. D. Borrmann, "Umsetzung von Diketen mit Alkoholen, Phenolen und Mercaptanen" im Houben-Weyls "Methoden der Organischen Chemie", Vol. VII/4, 230 ff. (1968)].

Die Enamine der allgemeinen Formel (V) sind neu. Sie werden nach bekannten Methoden hergestellt [vgl. z.B. A. C. Cope, J. Am. Chem. Soc. 67, 1047 (1945)].

Die Knoevenagel-Kondensationsprodukte (Ylidenverbindungen) der allgemeinen Formel (VII) sind neu. Sie werden nach bekannten Methoden hergestellt [vgl. z.B. G. Jones, Organic Reactions XV, 204 (1967)].

Dis als Ausgangsstoffe eingesetzten 1,4-Dihydropyridincarbonsäureester (IX) sind bekannt oder können nach bekannten Methoden hergestellt werden [vgl. DE 32 06 671, EP 00 71 819].

Das als Ausgangsstoff eingesetzte Anilin (X) ist bekannt oder kann nach literaturbekannten Methoden hergestellt werden (vgl. DOS 2 165 260; 2 847 237 und 2 401 665).

Die als Ausgangsstoffe eingesetzten Carbonsäuren (XI) sind bekannt oder können nach literaturbekannten Methoden hergestellt werden [vgl. DE 22 06 671].

Die als Ausgangsstoffe eingesetzten Aminoalkohole der Formel (XII) sind neu und können nach literaturbekannten Methoden hergestellt werden [vgl. Organikum, S. 191, VEB Deutscher Verlag der Wissenschaften Berlin 1963].

Als Lösemittel für die Verfahren A und B können Wasser oder alle inerten organischen Lösemittel verwendet werden, die sich unter den Reaktionsbedingungen nicht verändern. Hierzu gehören bevorzugt Alkohole wie Methanol, Ethanol, Propanol, Isopropanol, oder Ether wie Diethylether, Dioxan oder Tetrahydrofuran, oder Dimethylformamid, Dimethylsulfoxid, Acetonitril, Eisessig, Essigester, Hexamethylphosphorsäuretriamid, oder Kohlenwasserstoffe wie Benzol, Toluol oder Xylol. Ebenso können Gemische der

genannten Lösemittel eingesetzt werden.

Die Reaktionstemperaturen für die Verfahren A und B können in einem größeren Bereich variiert werden. Im allgemeinen arbeitet man in einem Bereich von +10° C bis +200° C, bevorzugt von +20° C bis 140° C insbesondere bis zur Siedetemperatur des verwendeten Lösemittels.

Die Umsetzungen können bei Normaldruck aber auch bei erhöhtem oder erniedrigtem Druck durchgeführt werden. Im allgemeinen arbeitet man bei Normaldruck.

Bei der Durchführung der erfindungsgemäßen Verfahren A und B ist das Verhältnis der an der Reaktion beteiligten Stoffe beliebig. Im allgemeinen arbeitet man mit equimolaren Mengen der Reaktanden. Bei Verfahren A und B hat es sich als zweckmäßig erwiesen, Nitroaceton bzw. das Nitroaceton-Ammoniak-Additionsprodukt im bis zu 20-fachen, bevorzugt bis zu 10-fachen Überschuß einzusetzen.

Die Durchführung der erfindungsgemäßen Verfahrensvariante C lehnt sich an die literaturbekannte Methodik zur Alkylierung von Aminen an. Dabei wird der aktivierte Alkylester IX in Gegenwart einer Hilfsbase mit dem Anilin X umgesetzt. Als Hilfsbasen seien beispielhaft angeführt: Trialkylamine wie Triethylamin, Tributylamin, Dimethylbenzylamin, Methylhexyl-isopropylamin sowie DBU, DBN oder Natriumbistrimethylsilylamid.

Die Durchführung der erfindungsgemäßen Verfahrensvariante D lehnt sich an die literaturbekannte Methodik zur Veresterung von Carbonsäuren an. Dabei wird die Carbonsäure zunächst in eine aktivierte Form wie z.B. das Säurechlorid oder das Imidazolid überführt, die entweder als solche isoliert und in einem zweiten Reaktionsschritt umgesetzt wird oder die in situ direkt zu den erfindungsgemäßen Verbindungen alkanolysiert wird. Als aktivierende Agentien seien neben den anorganischen Halogeniden wie Thionylchlorid, Phosphortrichlorid oder Phosphorpentachlorid auch Carbonyldiimidazoyl, Carbodiimide wie Dicyclohexylcarbodiimid oder 1-Cyclohexl-3-[2-(N-methyl-morpholino)ethyl]carbodiimid-p-toluolsulfonat oder N-Hydroxyphthalimid oder N-Hydroxy-benzotriazol in Gegenwart von Dicyclohexylcarbodiimid beispielhaft erwähnt. Naturgemäß lassen sich die 1,4-Dihydropyridinmonocarbonsäuren auch in Salze überführen, die mit Substraten der allgemeinen Formel (XIII)

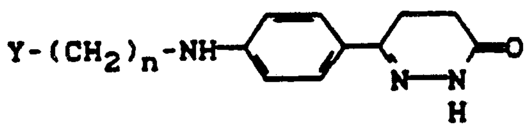

(XIII)

in welcher

n die angegebene Bedeutung hat

und

Y - eine nucleofuge Gruppe wie beispielsweise Iodid oder Tosylat darstellt,

zu den erfindungsgemäßen Verbindungen umgesetzt werden können.

Als Verdünnungsmittel kommen alle inerten organischen Lösemittel in Frage. Hierzu gehören vorzugsweise Ether wie Dioxan, Tetrahydrofuran, Glykolmonomethylether oder Glykoldimethylether, halogenierte Kohlenwasserstoffe wie Dichlormethan oder Trichlormethan, Dimethylformamid, Dimethylsulfoxid, Acetonitril, Pyridin und Hexamethylphosphorsäuretriamid. Isoliert man die aktivierten Zwischenstufen der 1,4-Dihydromonocarbonsäuren, so können der Alkohol der Formel (XII) auch allein als Verdünnungsmittel eingesetzt werden.

Die Alkanolyse wird zweckmäßigerweise durch Zugabe katalytischer oder equimolarer Mengen eines basischen Hilfsstoffs beschleunigt.

Bei der Durchführung der erfindungsgemäßen Verfahren C und D ist das Verhältnis der an der Reaktion beteiligten Stoffe beliebig. Im allgemeinen arbeitet man mit equimolaren Mengen der Reaktanden.

Die Reaktionstemperaturen können in einem größeren Bereich variiert werden. Im allgemeinen arbeitet man zwischen +10° C und +200° C, insbesondere zwischen +20° C und +150° C, vorzugsweise jedoch bei der Siedetemperatur des jeweiligen Lösemittels.

Die Umsetzung kann bei Normaldruck, aber auch bei erhöhtem Druck durchgeführt werden. Im allgemeinen arbeitet man unter Normaldruck.

Die erfindungsgemäßen Verbindungen zeigen ein nicht vorhersehbares, wertvolles pharmakologisches Wirkspektrum. Sie beeinflussen die Kontraktionskraft des Herzens und den Tonus der glatten Muskulatur. Sie können deshalb in Arzneimitteln zur Beeinflussung des pathologisch veränderten Blutdrucks, als Koronartherapeutika und zur Behandlung der Herzinsuffizienz, eingesetzt werden. Darüber hinaus können sie zur Behandlung von Herzrhythmusstörungen, zur Senkung des Blutzuckers, zur Abschwellung von Schleimhäuten und zur Beeinflussung des Salz- und Flüssigkeitshaushaltes verwendet werden.

Die Herz- und Gefäßwirkungen wurden am isoliert perfundierten Herzen des Meerschweinchens gefunden.

Dazu werden die Herzen von 250 bis 350 g schweren Meerschweinchen verwendet. Die Tiere werden mit einem Schlag auf den Kopf getötet, der Thorax geöffnet, und in die freipräparierte Aorta eine Metallkanüle eingebunden. Das Herz wird mit den Lungen aus dem Thorax herausgetrennt und über eine Aortenkanüle an die Perfusionsapparatur bei laufender Perfusion angeschlossen. Die Lungen werden an den Lungenwurzeln abgetrennt. Als Perfusionsmedium dient eine Krebs-Henseleit-Lösung (1) (118,5 mmol/l NaCl, 4,75 mmol/l KCl, 1,19 mmol/l $KH_2PO_4$, 1,19 mmol/l $MgSO_4$, 25 mmol/l $NaHCO_3$, 0,013 mmol/l $Na_2EDTA$), deren $CaCl_2$-Gehalt 1,2 mmol/l beträgt. Als energielieferndes Substrat werden 10 mmol/l Glucose zugesetzt, vor der Perfusion wird die Lösung partikelfrei filtriert. Die Lösung wird mit Carbogen (95% $O_2$, 5% $CO_2$) zur Aufrechterhaltung des pH-Wertes 7,4 begast. Die Herzen werden mit konstantem Fluß (10 ml/min) bei 32° C mittels einer Rollenquetschpumpe perfundiert.

Zur Messung der Herzfunktion wird ein flüssigkeitsgefüllter Latexballon, der über eine Flüssigkeitssäule mit einem Druckaufnehmer verbunden ist, durch den linken Vorhof in den linken Ventrikel eingeführt, und die isovolumetrischen Kontraktionen auf einem Schnellschreiber registriert (Opie, L., J. Physiol. 180 (1965), 529 -541). Der Perfusionsdruck wird mittels eines Druckaufnehmers, der vor dem Herzen mit dem Perfusionssystem in Verbindung steht, registiert. Unter diesen Bedingungen zeigt eine Senkung des Perfusionsdrucks eine Koronardilatation, eine Zu- bzw. Abnahme der linksventrikulären Kontraktionsamplitude einen Anstieg bzw. eine Senkung der Herzkontraktilität an. Die erfindungsgemäßen Verbindungen werden in geeigneten Verdünnungen in das Perfusionsmedium kurz vor dem isolierten Herzen infundiert.

Die folgenden Werte zeigen beispielhaft den Effekt der erfindungsgmäßen Verbindungen am isoliert perfundierten Meerschweinchenherzen, ausgedrückt als prozentuale Differenz gegenüber dem gleich 100% gesetzten Ausgangswert.

| Bsp.-Nr. | Konzentration (g/l) | %-Änderung der Kontraktionskraft | %-Änderung des Perfusionsdrucks |
|---|---|---|---|
| 5 | $10^{-6}$ | + 3% | -17% |
| 7 | $10^{-6}$ | + 9% | -17% |
| 8 | $10^{-6}$ | + 16% | -27% |
| 9 | $10^{-6}$ | + 40% | -26% |

Darüberhinaus wirken die erfindungsgemäßen Stoffe der allgemeinen Formel (I) als Hemmer/Stimulatoren von enzymatischen Reaktionen im Rahmen des Arachidonsäurestoffwechsels. Derartige Substanzen sind zur Verhütung und Behandlung von Erkrankungen der Atemwege wie Emphyse, Schocklunge, pulmonaler Hypertonie, Ödem, Thrombose und Thromboembolien, Ischämien (periphere, coronare, cerebrale Durchblutungsstörungen), Herz- und Hirninfarkte, Herzrhythmusstörungen, Angina pectoris, Hypertonie sowie Arteriosklerose geeignet. Bevorzugt wirken die erfindungsgemäßen Stoffe thrombozytenaggregationshemmend.

Zur Bestimmung der thrombozytenaggregationshemmenden Wirkung wurde Blut von gesunden Probanden beiderlei Geschlechts verwendet. Als Antikoagulans wurden einem Teil 3,8%iger wäßriger Natriumzitratlösung 9 Teile Blut zugemischt. Mittels Zentrifugation erhält man aus diesem Blut plättchenreiches Zitratplasma (PRP) (Jürgens/Beller, Klinische Methoden der Blutgerinnungsanalyse; Thieme Verlag, Stuttgart, 1959).

Für diese Untersuchungen wurden 0,8 ml PRP und 0,1 ml der Wirkstofflösung bei 37° C im Wasserbad vorinkubiert. Anschließend wurde die Thrombozytenaggregation nach der turbidometrischen Methode (Born, G.V.R., J. Physiol. (London), 162, 67, 1962) im Aggregometer bei 37° C bestimmt (Therapeutische Berichte 47, 80-86, 1975). Hierzu wurde die vorinkubierte Probe mit 0,1 ml Kollagen, einem aggregationsauslösenden Agens, versetzt. Die Veränderung der optischen Dichte in der Probe des PRP wurde während einer Zeitdauer von 4 Minuten aufgezeichnet und der Ausschlag nach 4 Minuten bestimmt. Hierzu wird die prozentuale Hemmung gegenüber der Kontrolle errechnet.

Als Grenzkonzentration wird die minimal effektive Konzentration angegeben, d.h. die Konzentration eine Hemmung von mindestens 25 % bewirkt.

Die erfindungsgemäßen Substanzen hemmen ab 3 µg/ml die kollageninduzierte Thrombozytenaggregation.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbbei soll die therapeutisch wirksame Verbindung jeweils in einer Konzentration von etwa 0,5 bis 90-Gew.-% der

Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt: Wasser, nicht-toxische organische Lösungsmittel, wie Paraffine (z.B. Erdölfraktionen), pflanzliche Öle (z.B. Erdnuß/Sesamöl), Alkohole (z.b: Ethylalkohol, Glycerin), Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester, Polyoxyethylen-Fettalkohol-Ether, Alkylsulfonate und Arylsulfonate), Dispergiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten.Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0.01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament , der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

## Herstellungsbeispiele

### Beispiel 1

N-(6-Hydroxyhexyl)-4-(6-oxo-1,4,5,6-tetrahydropyridazin-3-yl)-anilin

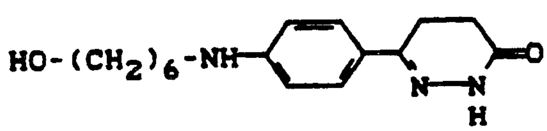

1,65 g Natriumhydrid werden in 50 ml Dimethylformamid vorgelegt. Portionsweise gibt man 9,45 g 4-(6-Oxo-1,4,5,6-tetrahydropyridazin-3-yl)anilin zu. Dazu tropft man die Lösung von 5,9 g 6-Bromhexyl-tetrahydropyranylether. Es wird 2 Stunden bei Raumtemperatur gerührt, dann auf Wasser gegossen. Der Niederschlag wird abgesaugt und mit Wasser gewaschen, dann in Methanol gelöst und unter Zugabe von 2 g sulfonsaurem Ionenaustauscher 8 Stunden zum Rückfluß erhitzt. Der Eindampfrückstand wird nach Abfiltrieren des Ionenaustauschers roh weiterverarbeitert.

Beispiel 2

Acetessigsäure-6-[4-(6-oxo-1,4,5,6-tetrahydropyridazin-3-yl)anilino]hexylester

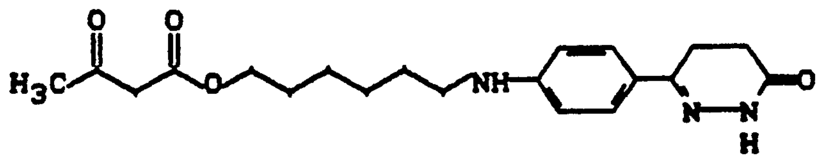

12,8 Hexanolderivat aus Beispiel 1 wird in 250 ml Tetrahydrofuran zum Rückfluß erhitzt. Es werden 18 g Diketen in 20 ml Tetrahydrofuran zugetropft. Man erhitzt noch 2 Stunden zum Rückfluß, destilliert das Lösemittel im Vakuum ab und kristallisiert den Rückstand aus wenig Isopropanol.
Schmp.: 125° C
Ausbeute: 10,2 g (62% der Theorie)

Beispiel 3

3-Aminocrotonsäure-6-[4-(6-oxo-1,4,5,6-tetrahydropyridazin-3-yl)anilino]hexylester

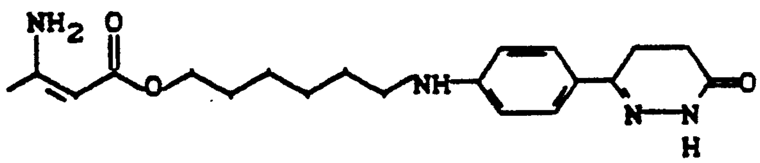

5,5 g Acetessigester aus Beispiel 2 werden in 150 ml Tetrahydrofuran mit 0,2 g p-Toluolsulfonsäure zum Rückfluß erhitzt. Dabei wird über 6 Stunden Ammoniak eingeleitet. Nach dem Abkühlen wird eingeengt und der Rückstand aus Isopropanol umkristallisiert.
Schmp.: 4,8 g (87% der Theorie)

Beispiel 4

3-Aminocrotonsäure-3-[4-(6-oxo-1,4,5,6-tetrahydropyridazin-3-yl)anilino]propylester

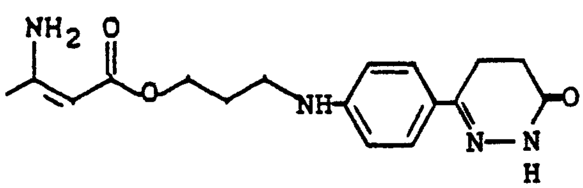

Die Titelverbindung wird analog Beispiel 3 aus N-(3-Hydroxypropyl)-4-(6-oxo-1,4,5,6-tetrahydropyridazin-3-yl)anilin hergestellt.

Beispiel 5

3-Oxo-1-(2-trifluormethylphenyl)-but-1-en-2-carbonsäure-3-[4-(6-oxo-1,4,5,6-tetrahydropyridazin-3-yl)anilino]-propylester

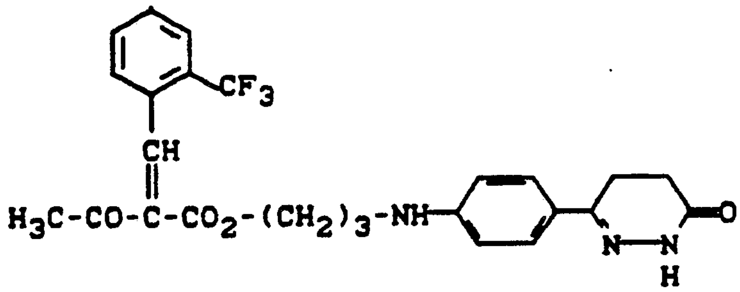

30 mmol 1-Butyliminomethyl-2-trifluormethyl-benzol und 30 mmol Acetessigsäure-3-[4-(6-oxo-1,4,5,6-tetrahydropyridazin-3-yl)anilino]propylester werden in 25 ml Acetanhydrid 24 Stunden bei Raumtemperatur gerührt, anschließend gießt man auf Wasser, dekantiert die wäßrige Phase und digeriert den Rückstand mit etwas Isopropanol.
Ausbeute: 68% der Theorie
Schmp.: 136 °C

Beispiel 6

1,4-Dihydro-2,6-dimethyl-5-nitro-4-(2-trifluormethylphenyl)-pyridin-3-carbonsäure-6-[4-(6-oxo-1,4,5,6-tetrahydropyridazin-3-yl)anilino]hexylester

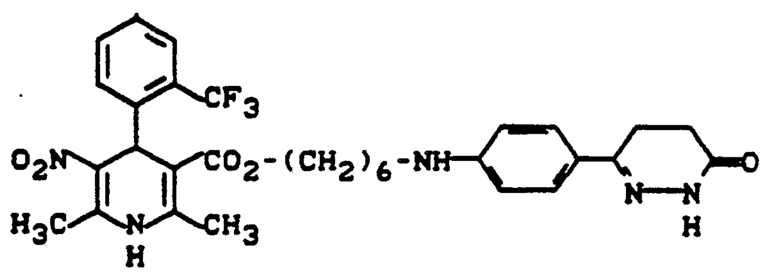

Verfahren A

10 mmol 2-Nitro-1-(2-trifluormethylphenyl)-but-1-en-3-on und 10 mmol 3-Aminocrotonsäure-6-[4-(6-oxo-1,4,5,6-tetrahydropyridazin-3-yl)anilino]hexylester werden in 30 ml Isopropanol 4 Stunden zum Rückfluß erhitzt. Beim Abkühlen kristallisiert die Verbindung aus und wird aus Isopropanol umkristallisiert.
Ausbeute: 4,1 g (80% der Theorie)
Schmp.: 140 °C (Zers.)

Verfahren C

10 mmol 1,4-Dihydro-2,6-dimethyl-5-nitro-4-(2-trifluormethylphenyl)-pyridin-3-carbonsäure-6-brom-hexylester und 10 mmol 4-(6-Oxo-1,4,5,6-tetrahydropyridazin-3-yl)anilin und 5 mmol DBN werden 10 Stunden in Isopropanol zum Rückfluß erhitzt. Nach dem Abkühlen wird abfiltriert und der Eindampfrückstand an Kieselgel mit Chloroform und 10% Methanol chromatographiert.
Schmp.: 140 °C (Zers.)
Ausbeute: 32% der Theorie

Beispiel 7 (Variante B mit Kondensationsprodukt)

1,4-Dihydro-2,6-dimethyl-5-nitro-4-(2-trifluormethylphenyl)-pyridin-3-carbonsäure-6-[4-(6-oxo-1,4,5,6-tetrahydropyridazin-3-yl)anilino]propylester

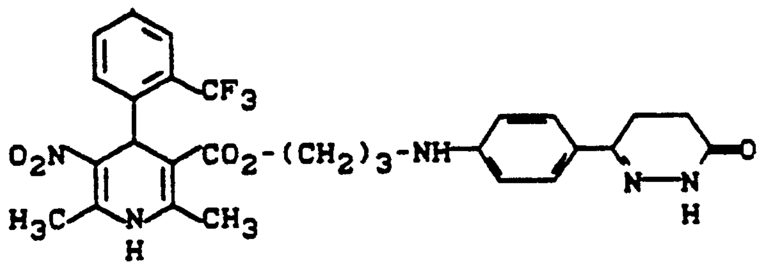

10 mmol Benzylidenverbindung aus Beispiel 5 werden in 30 ml Isopropanol bei 60° C portionsweise mit 30 mmol Ammoniak-Nitroaceton-Additionsprodukt versetzt. Es wird 4 Stunden nachgerührt. Das produkt wird durch Säulenchromatographie am Kieselgel mit Chloroform plus 10 % Methanol gereinigt.
Ausbeute: 26 % der Theorie
Schmp.: 182° C

Beispiel 8 (Variante A mit Kondensationsprodukt)

1,4-Dihydro-2,6-dimethyl-5-nitro-4-(3-nitrophenyl)-pyridin-3-carbonsäure-3-[4-(6-oxo-1,4,5,6-tetrahydropyridazin-3-yl)anilino]propylester

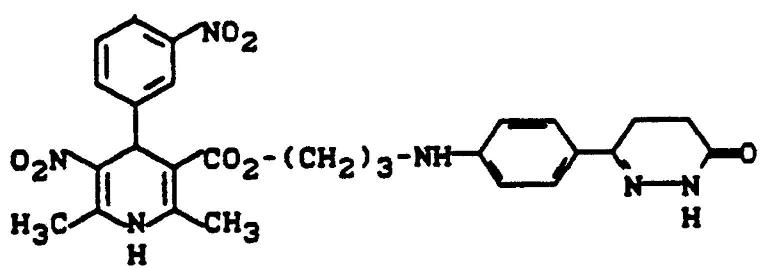

10 mmol 2-Nitro-1-(3-nitrophenyl)-but-1-en-3-on und 10 mmol 3-Aminocrotonsäure-3-[4-(6-oxo-1,4,5,6-tetrahydropyridazin-3-yl)anilino]propylester werden 6 Stunden in 30 ml Isopropanol auf 60° C erhitzt. Nach Abdestillieren des Lösemittels wird der Rückstand am Kieselgel mit Chloroform plus 10% Methanol chromatographiert.
Ausbeute: 32% der Theorie
Schmp.: 166° C

Beispiel 9 (Variante A mit Kondensationsprodukt)

4-(2-Benzyloxyphenyl)-1,4-dihydro-2,6-dimethyl-5-nitropyridin-3-carbonsäure-3-[4-(6-oxo-1,4,5,6-tetrahydropyridazin-3-yl)anilino]propylester

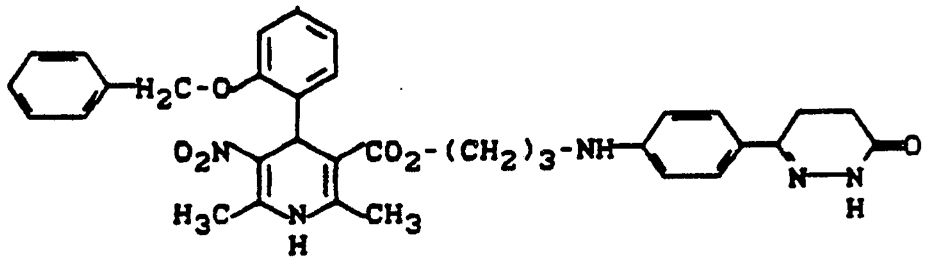

Die Titelverbindung wird analog Beispiel 8 aus 1-(2-Benzyloxyphenyl-2-nitro-but-1-en-3-on und 3-Aminocrotonsäure-3-[4-(6-oxo-1,4,5,6-tetrahydropyridazin-3-yl)anilino]propylester hergestellt.
Ausbeute: 29% der Theorie
Schmp.: Harz

Beispiel 10 (Variante A mit Kondensationsprodukt)

1,4-Dihydro-2,6-dimethyl-5-nitro-4-(2-trifluormethylphenyl)-pyridin-3-carbonsäure-6-bromhexylester

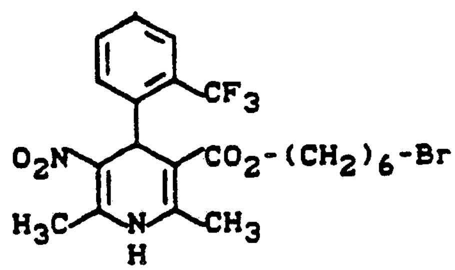

50 mmol 2-Nitro-1-(2-trifluormethylphenyl)-but-1-en-3-on und 50 mmol 3-Aminocrotonsäure-5-bromhexylester werden 6 Stunden in Isopropanol auf 60° C erhitzt. Nach teilweisem Abdampfen des Lösemittels kristallisiert das Produkt.
Schmp.: 117° C
Ausbeute: 42% der Theorie

Beispiel 11 (Variante A)

4-(2-Chlorphenyl)-1,4-dihydro-2,6-dimethyl-5-nitro-pyridin-3-carbonsäure-4-[4-(6-oxo-1,4,5,6-tetrahydropyridazin-3-yl)anilino]butylester

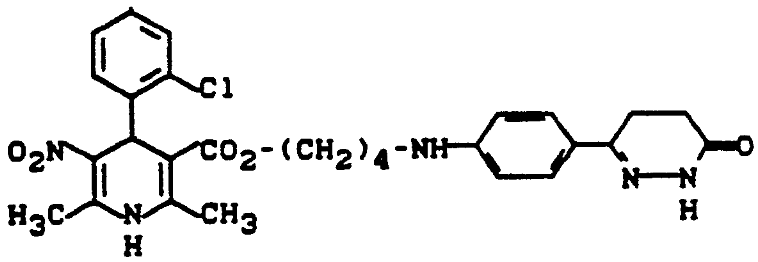

Die Titelverbindung wird analog Beispiel 5 aus 1-(2-Chlorphenyl)-2-nitro-but-1-en-3-on und 3-Aminocrotonsäure-4-[4-(6-oxo-1,4,5,6-tetrahydropyridazin-3-yl)-anilino]butylester erhalten.
Schmp.: 143 °C (Zers.)

Beispiel 12 (Variante A)

1,4-Dihydro-2,6-dimethyl-4-(2-methoxyphenyl)-5-nitropyridin-3-carbonsäure-6-[4-(6-oxo-1,4,5,6-tetrahydropyridazin-3-yl)anilino]hexylester

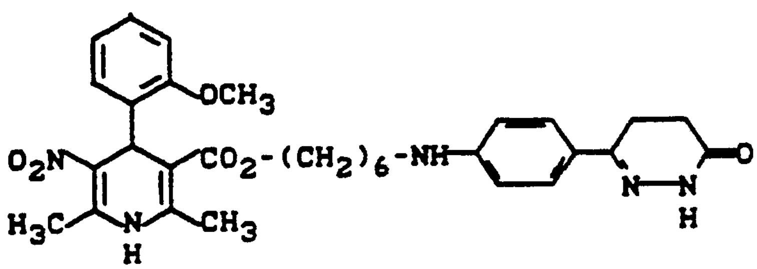

20 mmol 2-Methoxybenzaldehyd und 20 mmol 3-Aminocrotonsäure-6-[4-(6-oxo-1,4,5,6-tetrahydropyrida zin-3-yl)anilino]hexylester werden in 50 ml Isopropanol bei 40 °C portionsweise mit 50 mmol Nitroaceton-Ammoniak-Additionsverbindung versetzt. Man läßt 4 h nachrühren und dampft ein. Der Rückstand wird an Kieselgel mit Chloroform plus 10% Methanol chromatographiert.
Ausbeute: 26% der Theorie
Schmp.: Harz

**Ansprüche**

1. Nitro-dihydropyridine der allgemeinen Formel (I)

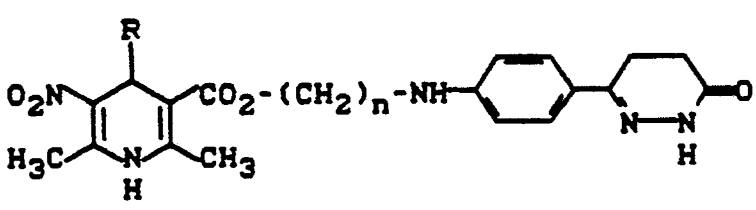 (I),

in welcher

R- für Aryl mit 6 bis 12 Kohlenstoffatomen steht, das bis zu 4-fach gleich oder verschieden substituiert sein kann durch Halogen, Nitro, Cyano, Alkyl, Alkoxy, Alkylthio mit jeweils bis zu 6 Kohlenstoffatomen, Trifluormethyl, Trifluormethoxy, Difluormethoxy, Trifluormethylthio, Difluormethylendioxy, Trifluorethylendioxy, Tetrafluorethylendioxy oder durch gegebenenfalls durch Halogen, Nitro, Cyano, Trifluormethyl, Alkyl mit bis zu 4 Kohlenstoffatomen oder Alkoxy mit bis zu 4 Kohlenstoffatomen substituiertes Benzyl, Benzyloxy oder Benzylthio, oder

- für einen gegebenenfalls durch Halogen, Phenyl oder Alkyl mit bis zu 4 Kohlenstoffatomen substituierten Heterocyclus aus der Reihe Pyrryl, Thienyl, Furyl, Pyrazolyl, Imidazolyl, Oxazolyl, Thiazolyl, Pyridyl, Pyrimidyl, Pyridazinyl, Chinolyl, Benzoxadiazolyl, Chromenyl oder Thiochromenyl steht

und

n- für eine Zahl 1 bis 12 steht

und deren physiologisch unbedenkliche Salze.

2. Verbindungen der allgemeinen Formel (I) gemäß Anspruch 1, in welcher

R- für Phenyl oder Naphthyl steht, das bis zu 3-fach gleich oder verschieden substituiert sein kann durch Fluor, Chlor, Brom, Nitro, Cyano, Alkyl, Alkoxy, Alkylthio mit jeweils bis zu 4 Kohlenstoffatomen, Trifluormethyl, Trifluormethoxy, Trifluormethylthio, durch gegebenenfalls durch Nitro, Fluor, Chlor, Brom, Trifluormethyl, Methyl oder Methoxy substituiertes Benzyloxy oder Benzylthio, oder

- für einen gegebenenfalls durch Methyl, Fluor, Chlor, Brom oder Phenyl substituierten Heterocyclus aus der Reihe Thienyl, Furyl, Pyridyl, Pyrimidyl, Chinolyl, Benzoxadiazolyl oder Thiochromenyl steht

und

n - für eine Zahl 1 bis 10 steht

und deren physiologisch unbedenkliche Salze.

3. Verbindungen der allgemeinen Formel (I) gemäß Anspruch 1, in welcher

R - für Phenyl steht, das bis zu 2-fach gleich oder verschieden substituiert sein kann durch Fluor, Chlor, Nitro, Cyano, Alkyl, Alkoxy mit jeweils bis zu 4 Kohlenstoffatomen, Trifluormethyl, Benzyloxy oder Benzylthio, oder

- für Thienyl, Furyl, Pyridyl, Benzoxadiazolyl steht, oder

- für einen Heterocyclus der Formel

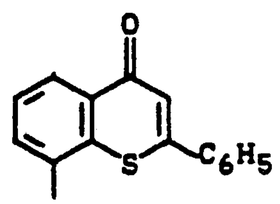

steht,

und

n - für eine Zahl 2 bis 8 steht

und deren physiologisch unbedenkliche Salze.

4. Verfahren zur Herstellung von Nitro-dihydropyridinen der allgemeinen Formel (I)

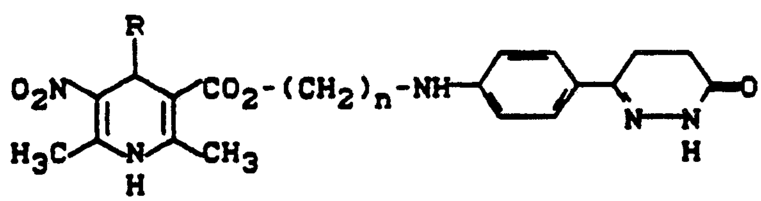

(I),

in welcher

R - für Aryl mit 6 bis 12 Kohlenstoffatomen steht, das bis zu 4-fach gleich oder verschieden substituiert sein kann durch Halogen, Nitro, Cyano, Alkyl, Alkoxy, Alkylthio mit jeweils bis zu 6 Kohlenstoffatomen, Trifluormethyl, Trifluormethoxy, Difluormethoxy, Trifluormethylthio, Difluormethylendioxy, Trifluorethylendioxy, Tetrafluorethylendioxy oder durch gegebenenfalls durch Halogen, Nitro, Cyano, Trifluormethyl, Alkyl mit bis zu 4 Kohlenstoffatomen oder Alkoxy mit bis zu 4 Kohlenstoffatomen substituiertes Benzyl, Benzyloxy oder Benzylthio, oder

- für einen gegebenenfalls durch Halogen, Phenyl oder Alkyl mit bis zu 4 Kohlenstoffatomen substituierten

Heterocyclus aus der Reihe Pyrryl, Thienyl, Furyl, Pyrazolyl, Imidazolyl, Oxazolyl, Thiazolyl, Pyridyl, Pyrimidyl, Pyridazinyl, Chinolyl, Benzoxadiazolyl, Chromenyl oder Thiochromenyl steht
und
n - für eine Zahl 1 bis 12 steht
und deren physiologisch unbedenkliche Salze, dadurch gekennzeichnet, daß man

[A] Aldehyde der allgemeinen Formel (II)

R-CHO (II)

in welcher
R die oben angegebene Bedeutung hat
und Nitroaceton der Formel (III)

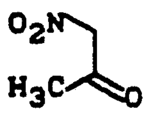

(III)

oder deren Knoevenagel-Kondensationsprodukte (Ylidenverbindungen) der allgemeinen Formel (IV)

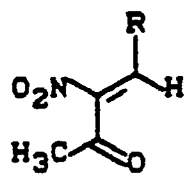

(IV)

in welcher
R die oben angegebene Bedeutung hat,
mit Enaminen der allgemeinen Formel (V)

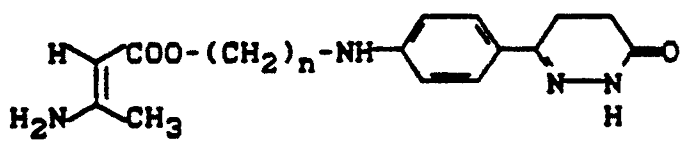

(V)

in welcher
n die oben angegebene Bedeutung hat,
in Gegenwart inerter Lösemittel umsetzt,
oder daß man

[B] Aldehyde der allgemeinen Formel (II) und Ketone der allgemeinen Formel (VI)

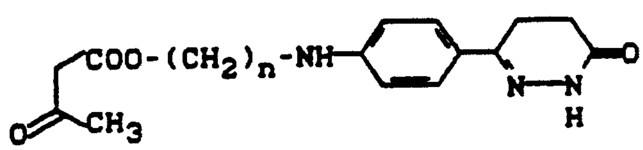

(VI)

in welcher
n die oben angegebene Bedeutung hat,
oder deren Knoevenagel-Kondensationsprodukte (Ylidenverbindungen) der allgemeinen Formel (VII)

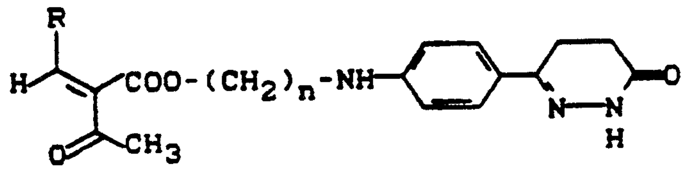

(VII)

in welcher
R und n die oben angegebene Bedeutung haben,
mit dem Enamin (Nitroaceton/Ammoniak-Additions-verbindung) der Formel (VIII),

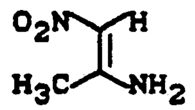

(VIII)

in Gegenwart inerter Lösemittel umsetzt,
oder daß man

[C] 1,4-Dihydropyridincarbonsäureester der allgemeinen Formel (IX)

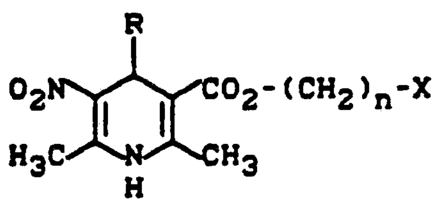

(IX)

in welcher
R und n die oben angegebene Bedeutung haben
und
X - eine nucleofuge Gruppe wie beispielsweise Bromid, Iodid, Mesylat, Triflat oder Tosylat darstellt,
mit dem Anilin der Formel (X)

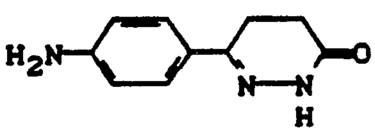

(X)

in Gegenwart einer Hiflsbase in einem inerten Lösemittel umsetzt,
oder daß man

[D] 1,4-Dihydropyridincarbonsäuren der allgemeinen Formel (XI)

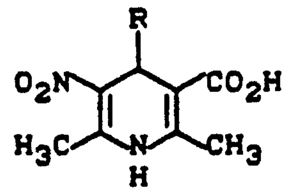
(XI)

in welcher
R die angegebene Bedeutung hat,
nach üblichen Methoden der Veresterung von Carbonsäuren (z.B. über das Carbonsäurechlorid oder -imidazolid oder in Gegenwart von Dicyclohexylcarbodiimid) mit dem Alkohol der Formel (XII)

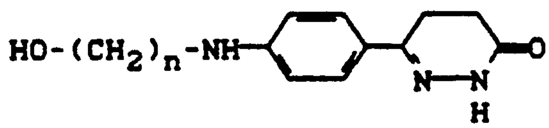
(XII)

in welcher
n die angegebene Bedeutung hat,
verestert.

5. Enamine der allgemeinen Formel (V)

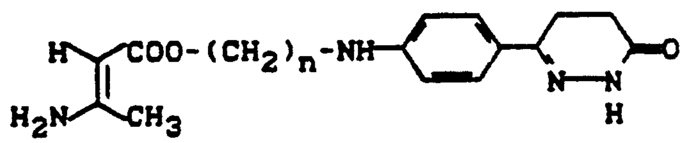
(V)

in welcher
n für eine Zahl von 1 bis 12 steht.

6. Verwendung von Enaminen der allgemeinen Formel (V) gemäß Anspruch 5 zur Herstellung von Verbindungen der allgemeinen Formel (I) gemäß Anspruch 1.

7. Ketone der allgemeinen Formel (VI)

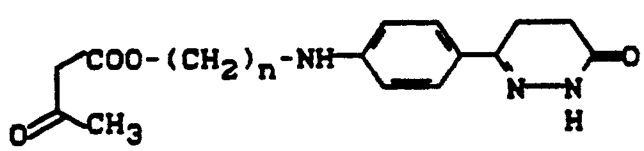
(VI)

in welcher
n für eine Zahl von 1 bis 12 steht und deren Knoevenagel-Kondensationsprodukte der allgemeinen Formel (VII)

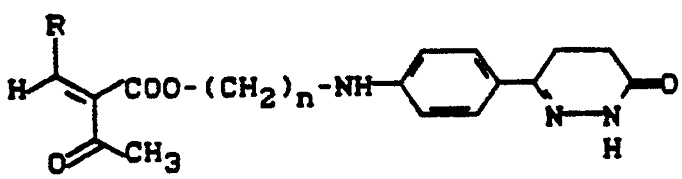
(VII)

in welcher
R- für Aryl mit 6 bis 12 Kohlenstoffatomen steht, das bis zu 4-fach gleich oder verschieden substituiert sein kann durch Halogen, Nitro, Cyano, Alkyl, Alkoxy, Alkylthio mit jeweils bis zu 6 Kohlenstoffatomen,

Trifluormethyl, Trifluormethoxy, Difluormethoxy, Trifluormethylthio, Difluormethylendioxy, Trifluorethylendioxy, Tetrafluorethylendioxy oder durch gegebenenfalls durch Halogen, Nitro, Cyano, Trifluormethyl, Alkyl mit bis zu 4 Kohlenstoffatomen oder Alkoxy mit bis zu 4 Kohlenstoffatomen substituiertes Benzyl, Benzyloxy oder Benzylthio, oder
- für einen gegebenenfalls durch Halogen, Phenyl oder Alkyl mit bis zu 4 Kohlenstoffatomen substituierten Heterocyclus aus der Reihe Pyrryl, Thienyl, Furyl, Pyrazolyl, Imidazolyl, Oxazolyl, Thiazolyl, Pyridyl, Pyrimidyl, Pyridazinyl, Chinolyl, Benzoxadiazolyl, Chromenyl oder Thiochromenyl steht
und
n - die oben angegebene Bedeutung hat.

8. Verwendung von Ketonen der allgemeinen Formel (VI) und ihren Knoevenagel-Kondensationsprodukten der allgemeinen Formel (VII) gemäß Anspruch 7 bei der Herstellung von erfindungsgemäßen Verbindungen der allgemeinen Formel (I) gemäß Anspruch 1.

9. Aminoalkohole der allgemeinen Formel (XII)

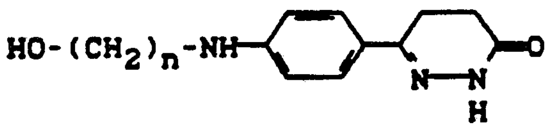

(XII)

in welcher
n für eine Zahl von 1 bis 12 steht.

10. Verwendung von Aminoalkoholen der allgemeinen Formel (XII) gemäß Anspruch 9 bei der Herstellung von Verbindungen der allgemeinen Formel (I) gemäß Anspruch 1.

11. Verbindungen der allgemeinen Formel (I) gemäß Anspruch 1 zur Verwendung bei der Bekämpfung von Kreislauferkrankungen und Thrombosen.

12. Arzneimittel enthaltend mindestens eine Verbindung der allgemeinen Formel (I) gemäß Anspruch 1 sowie gegebenenfalls weitere übliche Hilfs- und Trägerstoffe.

13. Verfahren zur Herstellung von Arzneimitteln, dadurch gekennzeichnet, daß man Verbindungen der allgemeinen Formel (I) gemäß Anspruch 1 gegebenenfalls unter Verwendung üblicher Hilfs- und Trägerstoffe in eine geeignete Applikationsform überführt.

14. Verwendung von Verbindungen der allgemeinen Formel (I) gemäß Anspruch 1 bei der Bekämpfung von Kreislauferkrankungen und Thrombosen.

Europäisches Patentamt

# EUROPÄISCHER TEILRECHERCHENBERICHT,
der nach Regel 45 des Europäischen Patentübereinkommens für das weitere Verfahren als europäischer Recherchenbericht gilt

Nummer der Anmeldung

EP 88 11 1484

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | EP-A-0 173 204 (BAYER AG)<br>* Ansprüche *<br>-- | 1-4, 12,13 | C 07 D 401/12<br>A 61 K 31/50<br>C 07 D 237/04<br>//A 61 K 31/445 |
| Y | EP-A-0 185 964 (BAYER AG)<br>* Seiten 1-9 *<br>-- | 1-4, 12,13 | |
| A | EP-A-0 174 654 (CIBA-GEIGY)<br>* Ansprüche *<br>-- | 1 | |
| D,A | EP-A-0 071 819 (BAYER AG)<br>* Ansprüche *<br>--------- | 1 | |

RECHERCHIERTE SACHGEBIETE (Int. Cl.4)

C 07 D 401/00
A 61 K 31/00
C 07 D 237/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-13

Unvollständig recherchierte Patentansprüche: 14

Nicht recherchierte Patentansprüche:

Grund für die Beschränkung der Recherche:

Verfahren zur chirurgischen oder therapeutischen Behandlung des menschlichen oder tierischen Körpers (Siehe Art. 52(4) des Europäischen Patentübereinkommens)

| Recherchenort | Abschlußdatum der Recherche | Prüfer |
|---|---|---|
| Den Haag | 05-12-1988 | BRIGHENTI |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument